# EUROPEAN PATENT APPLICATION

(11) **EP 1 797 906 A2**
(43) Date of publication of application: **20.06.2007**
(21) Application number: 06256361.4
(22) Date of filing: 14.12.2006
(51) Int. Cl.: A61L 17/10

(54) **High strength suture formed of UHMWPE and PBT**

(30) Priority: 14.12.2005 US 302130
(71) Applicant: Arthrex, Inc., Naples, Florida 34108-1945 (US)
(72) Inventor: Schmieding, John W., Naples Florida 34109 (US)
(74) Representative: Collins, John David

(57) **Abstract**

A high strength abrasion resistant surgical suture material with improved tie down characteristics. The suture features a multifilament cover formed of strands of ultra high molecular weight long chain polyethylene braided with polybutylene terephthalate. Selected fibers in the cover may be provided in a color contrasting with the other cover fibers to provide an identifiable trace. The cover surrounds a core formed of twisted strands of ultrahigh molecular weight polyethylene. The suture, provided in a #2 size, has the strength of #5 Ethibond, is ideally suited for most orthopedic procedures, and can be attached to a suture anchor or a curved needle.

## Description

The present invention relates to high strength surgical suture materials, and more particularly to braided suture blends of ultrahigh molecular weight polyethylene (UHMWPE) and polybutylene terephthalate (PBT).

Suture strength is an important consideration in any surgical suture material. One of the strongest materials currently formed into elongated strands is an ultrahigh molecular long chain weight polyethylene, typically used for fishing line and the like, which is sold under the trade names Dyneema or Spectra. This material is much stronger than ordinary surgical suture, however, it does not have acceptable knot tie down characteristics for use in surgical applications.

The present invention advantageously provides a high strength surgical suture material with improved tie down characteristics. The suture features a braided cover made of a blend of ultrahigh molecular weight long chain polyethylene (UHMWPE) fiber and a fiber of one or more long chain synthetic polymers, preferably polybutylene terephthalate (PBT). The ultrahigh molecular weight polyethylene provides strength. The polybutylene terephthalate provides improved tie down properties.

Handling properties of the high strength suture also are enhanced using various materials to coat the suture. In addition, strands of a contrasting color may be added to the braided threads to enhance visibility. The colored strands preferably are dyed filaments of PBT or nylon.

In a preferred embodiment, the suture includes a multifilament cover formed of ultrahigh molecular weight polyethylene fiber braided with polybutylene terephthalate. The cover surrounds a fiber core formed substantially or entirely of ultrahigh molecular weight polyethylene. The core preferably comprises 3 strands of ultrahigh molecular weight polyethylene, twisted at about 3 to 6 twists per inch.

The cover preferably comprises 8 strands of ultrahigh molecular weight polyethylene braided with 8 strands of polybutylene terephthalate.

Ultrahigh molecular weight polyethylene fibers suitable for use in one embodiment of the present invention are marketed under the Dyneema trademark by DSM, or alternatively under the Spectra trademark by Honeywell.

The suture of one embodiment of the present invention advantageously has the strength of Ethibond #5 suture, yet has the diameter, feel and tie ability of #2 suture. As a result, the suture of an embodiment of the present invention is ideal for most orthopedic procedures such as rotator cuff repair, achilles tendon repair, patellar tendon repair, ACL/PCL reconstruction, hip and shoulder reconstruction procedures, and replacement for suture used in or with suture anchors.

The suture can be uncoated, or coated with wax (beeswax, petroleum wax, polyethylene wax, or others), silicone (Dow Corning silicone fluid 202A or others), silicone rubbers (Nusil Med 2245, Nusil Med 2174 with a bonding catalyst, or others) PTFE (Teflon, Hostaflon, or others), PBA (polybutylate acid), ethyl cellulose (Filodel) or other coatings, to improve handleability, knot sliding, tissue passing, lubricity of the braid, knot security, or abrasion resistance, for example.

As an added advantage, a few trace threads having a contrasting color, preferably of a readily dyed fiber, such as nylon, in the cover aid surgeons in identifying the travel direction of the suture during surgery, particularly during arthroscopic operations. Providing the trace threads in a regularly repeating pattern is particularly useful, allowing the surgeon to distinguish different ends of lengths of suture, and determine the direction of travel of a moving length of suture. Of the more easily dyed fibers, nylon is preferred in that it accepts dye readily. PBT fibers are stronger, but do not take up dye as easily as nylon.

Other features and advantages of the present invention will become apparent from the following description of embodiment of the invention which refers to the accompanying drawings.

Fig. 1 is a copy of a scanning electron micrograph of a length of suture according to the present invention.

Fig. 2 is a schematic cross section of a length of suture according to the present invention.

Fig. 3 is an illustration of the suture of the present invention attached to a suture anchor.

Fig. 4A and 4B show the suture of the present invention attached to a half round, tapered needle.

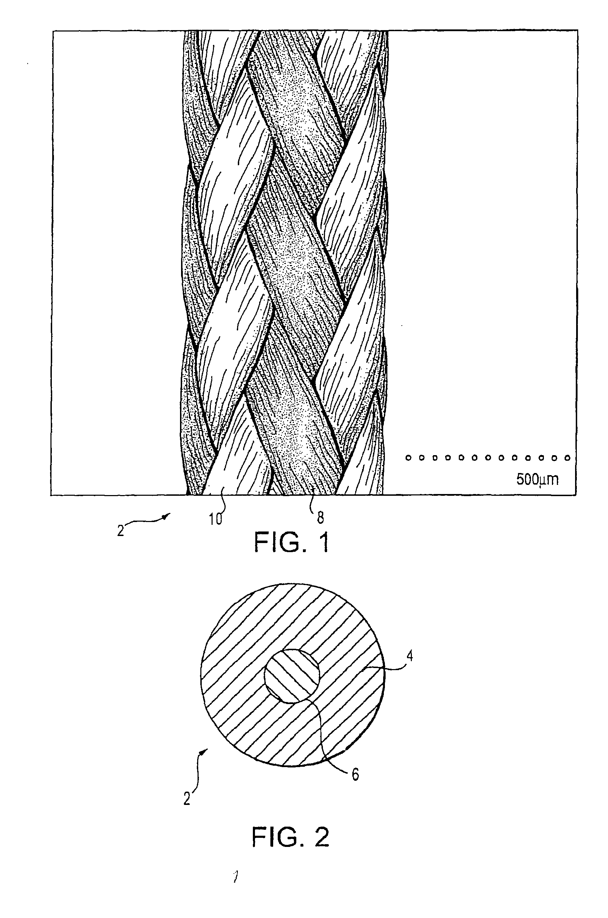

Referring to Fig. 1, a scanning electron micrograph of a length of suture 2 according to one embodiment of the present invention is shown. Suture 2 is made up of a cover 4 and a core 6 surrounded by the cover. See Fig. 2. Strands of ultrahigh molecular weight polyethylene (UHMWPE) 8, sold under the tradename Spectra or Dyneema, and strands of polybutylene terephthalate (PBT) 10 are braided together to form the cover 4. Core 6 is formed of twisted strands of UHMWPE.

UHMWPE strands 8 are substantially translucent or colorless. The PBT strands 10 are white (undyed). Optionally, one or more of the PBT strands or nylon strands (in place of respective PBT strands) having a contrasting color provide a trace in the suture. Due to the transparent nature of the UHMWPE, the suture takes on the color of PBT strands 10 and the trace strands, and thus appears to be white with a trace in the contrasting color. The trace strands, if included, are preferably provided in black. The black trace assists the surgeon in differentiating between suture strands with the trace and suture strands without the trace. The trace also assists the surgeon in identifying whether the suture is moving.

Details of the present invention will be described further below in connection with the following examples:
Example: USP Size 5 (EP size 7)

Made on a 16 carrier Hobourns machine, the yarns used in the braided cover are Dyneema Purity and PBT. The cover is formed using eight strands of 144 decitex Dyneema per carrier, braided with eight strands of 100 decitex PBT per carrier. The core is formed of three carriers of 144 decitex Dyneema braided at three to six twists per inch.

The example set forth above is for size 5 suture. The suture may also be made in USP size 2, in which case it is preferably formed of about 38% PBT, 62% UHMWPE. In the making of various sizes of the inventive suture, different decitex values can be used to achieve the required size and strength needed. In addition, smaller sizes may require manufacture on 12 carrier machines, for example. The very smallest sizes preferably are made without a core. Overall, the suture may range from 5% to 90% ultrahigh molecular weight polymer (preferably at least 40% of the fibers are ultrahigh molecular weight polymer), with the balance formed of PBT and/or PBT and nylon. The core preferably comprises 18% or greater of the total amount of filament.

The suture preferably is coated with wax (beeswax, petroleum wax, polyethylene wax, or others), silicone (Dow Corning silicone fluid 202A or others), silicone rubbers (Nusil Med 2245, Nusil Med 2174 with a bonding catalyst, or others) PTFE (Teflon, Hostaflon, or others), PBA (polybutylate acid), ethyl cellulose (Filodel) or other coatings, to improve lubricity of the braid, knot security, or abrasion resistance, for example.

The ultra high molecular weight (UHMW) polymer component of the present invention provides strength, and the PBT component is provided to improve tie ability and tie down characteristics.

According to an alternative embodiment of the present invention, a partially bioabsorbable suture is provided by blending a high strength material, such as UHMWPE fibers, with a bioabsorbable material, such as PLLA or one of the other polylactides, for example. Accordingly, a suture made with about 10% Spectra or Dyneema blended with absorbable fibers would provide greater strength than existing bioabsorbable suture with less stretch. Over time, 90% or more of the suture would absorb, leaving only a very small remnant of the knot. The absorbable suture can include coatings and colored traces as noted above for nonabsorbable suture.

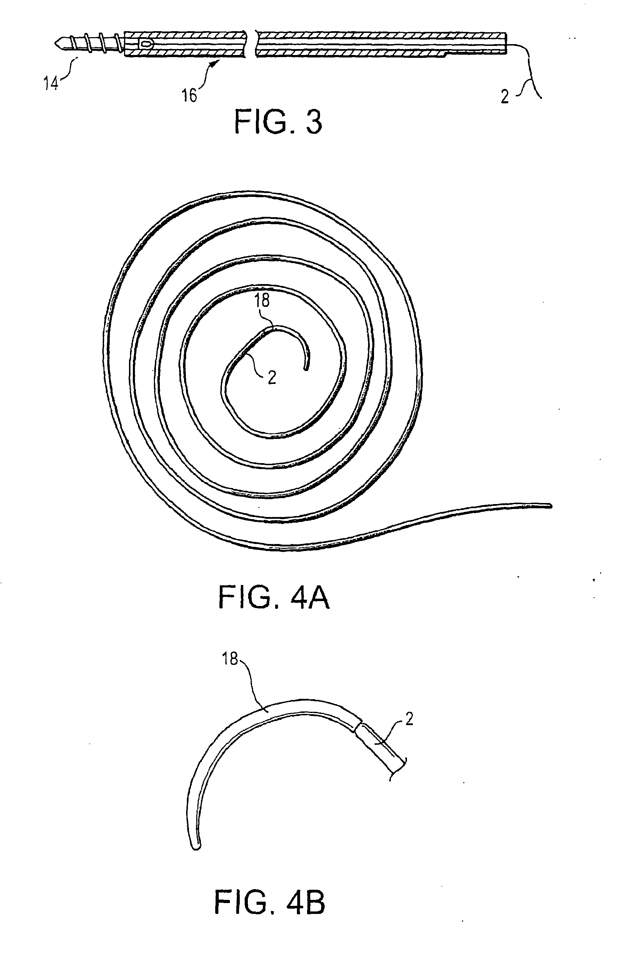

In one method of using the suture, the suture 2 is attached to a suture anchor 14 as shown in Fig. 3 (prepackaged sterile with an inserter 16), or is attached at one or both ends to a half round, tapered needle 18 as shown in Figs. 4A and 4B.

As mentioned above, one or more strands of a fiber in the blend can be provided in pre-dyed colors, e.g., black, to provide a trace. The trace threads enhance the ability to visually detect suture motion and the ability to differentiate between colored and uncolored suture strands.

Although the present invention has been described in relation to particular embodiments thereof, many other variations and modifications and other uses will become apparent to those skilled in the art.

## Claims

1. A suture strand suitable for use as a suture or ligature comprising a plurality of fibers of ultrahigh molecular weight polyethylene, and a plurality of fibers of polybutylene terephthalate, braided together.

2. The suture strand of claim 1, wherein the ultrahigh molecular weight polyethylene comprises at least 40% of the braided fibers.

3. The suture strand of claim 1, wherein the polybutylene terephthalate comprises less than about 40% of the braided fibers.

4. The suture strand of claim 1, further comprising a core of twisted fibers of ultrahigh molecular weight polyethylene surrounded by a cover comprising the plurality of braided fibers of ultrahigh molecular weight polyethylene and polybutylene terephthalate.

5. The suture strand of claim 4, wherein the core comprises about 18% or greater of the total amount of fibers.

6. The suture strand of claim 4, wherein the cover comprises less than about 82% of the total amount of fibers.

7. The suture strand of claim 4, further comprising a coating disposed on the cover.

8. The suture strand of claim 7, wherein the coating is selected from the group consisting of wax, silicone, silicone rubbers, PTFE, PBA, and ethyl cellulose.

9. A suture assembly comprising:
a suture comprising a plurality of fibers of ultrahigh molecular weight polyethylene and a plurality of fibers of polybutylene terephthalate, braided together; and
a suture anchor attached to the suture.

10. A suture assembly comprising:
a suture comprising a plurality of fibers of ultrahigh molecular weight polyethylene and a plurality of fibers of polybutylene terephthalate, braided together; and
a half round, tapered needle attached to one or both ends of the suture.

11. A suture strand suitable for use as a suture or ligature comprising a plurality of fibers of ultrahigh molecular weight polyethylene, and a plurality of fibers of polybutylene terephthalate, braided together, the suture stand being coated.

12. The suture strand of claim 11, wherein the coating is selected from the group consisting of wax, silicone, silicone rubbers, PTFE, PBA, and ethyl cellulose.
